(19) Europäisches Patentamt
European Patent Office
Office européen des brevets

(11) EP 0 873 289 B1

(12) EUROPÄISCHE PATENTSCHRIFT

(45) Veröffentlichungstag und Bekanntmachung des Hinweises auf die Patenterteilung:
**03.05.2000 Patentblatt 2000/18**

(21) Anmeldenummer: **96943892.8**

(22) Anmeldetag: **06.12.1996**

(51) Int Cl.[7]: **C07B 41/00**, C07C 41/54, C07C 41/08, C07C 45/26, C07D 319/10

(86) Internationale Anmeldenummer:
**PCT/EP96/05457**

(87) Internationale Veröffentlichungsnummer:
**WO 97/21648 (19.06.1997 Gazette 1997/26)**

# (54) VERFAHREN ZUR KATALYTISCHEN ADDITION VON NUCLEOPHILEN AN ALKINE ODER ALLENE

PROCESS FOR CATALYTIC ADDITION OF NUCLEOPHILES TO ALKINES OR ALLENES

PROCEDE D'ADDITION CATALYTIQUE DE NUCLEOPHILES ET D'ALCYNES OU D'ALLENES

(84) Benannte Vertragsstaaten:
**CH DE FR GB LI NL**

(30) Priorität: **13.12.1995 DE 19546610**

(43) Veröffentlichungstag der Anmeldung:
**28.10.1998 Patentblatt 1998/44**

(73) Patentinhaber: **BASF AKTIENGESELLSCHAFT
67056 Ludwigshafen (DE)**

(72) Erfinder:
- **SCHULZ, Michael
D-67067 Ludwigshafen (DE)**
- **TELES, Joaquim, Henrique
D-67059 Ludwigshafen (DE)**

(74) Vertreter: **Isenbruck, Günter, Dr.
Patent- und Rechtsanwälte,
Bardehle-Pagenberg-Dost-Altenburg-Geissler-Isenbruck
Theodor-Heuss-Anlage 12
68165 Mannheim (DE)**

(56) Entgegenhaltungen:
- **JOURNAL OF ORGANIC CHEMISTRY, Bd. 56, Nr. 11, 24.Mai 1991, EASTON US, Seiten 3729-3731, XP000204868 Y. FUKUDA ET AL: "Effective transformation of unactivated alkynes into ketones or acetals by means of Au(III) catalyst" in der Anmeldung erwähnt**
- **DATABASE WPI Section Ch, Week 9219 Derwent Publications Ltd., London, GB; Class B05, AN 92-156242 XP002027963 & JP 04 095 039 A ((KYOX) KYOWA YUKA) , 27.März 1992**
- **DATABASE WPI Section Ch, Week 9220 Derwent Publications Ltd., London, GB; Class B05, AN 92-163722 XP002027964 & JP 04 103 552 A ((KYOX) KYOWA YUKA) , 6.April 1992**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 873 289 B1

## Beschreibung

Die Erfindung betrifft ein für einen breiten Bereich von Alkinen und Allenen anwendbares Verfahren zur Addition von Nucleophilen an Alkine und Allene in Gegenwart eines zumindest teilweise in ionisierter Form vorliegenden Komplexes des einwertigen Golds.

Die Addition von Nucleophilen an Alkine wird durch Säuren, Basen oder Übergangsmetallkomplexe katalysiert (vgl. Houben-Weyl, Methoden der organischen Chemie, Bd 6/3, S. 233, S. 90, Bd. 5/2a, S. 738, Bd. 6/ld, S. 136 und Bd. 7/a, S. 816).

Die Säurekatalyse ist meistens auf die Addition von Nucleophilen an aktivierte, elektronenreiche Alkine (wie Acetylenether, R-C≡C-OR', Acetylenthioether, R-C≡C-SR' und Acetylenamine, $R\text{-}C{\equiv}C\text{-}NR'_2$) beschränkt.

Alkohole können basenkatalysiert (in Gegenwart von KOH oder Alkoholat) an nicht aktivierte Alkine addiert werden. Dies ist die Methode der Wahl für die Monoaddition von Alkoholen an Alkine unter Bildung von Enolethern.

Die Addition von Nucleophilen an Alkine wird auch durch Übergangsmetallkomplexe katalysiert. In der Regel handelt es sich um Komplexe von Metallen der Gruppen 11 und 12 (nach der derzeit gültigen JUPAC-Nomenklatur der anorganischen Chemie). Vereinzelt sind auch Rhodium-, Ruthenium-, Palladium- und Platinkatalysatoren eingesetzt worden.

Gemäß J.S. Reichert, H.H. Bailey, J.A. Nieuwland, J. Am. Chem. Soc., 45, 1552 (1923), und G.F. Hennion, J.A. Nieuwland, J. Am. Chem. Soc., 57, 2006 (1935), sind Quecksilber(II)verbindungen, meistens in Kombination mit einer Lewis- und einer Brönstedsäure, die aktivsten Katalysatoren für die Addition von Nucleophilen an Alkine. Mit solchen Hg(II)-Katalysatoren können Wasser, Alkohole und Carbonsäuren an Alkine addiert werden. Diese Katalysatoren sind sehr allgemein verwendbar, allerdings ist ihre Anwendungsbreite durch die Toxizität des Quecksilbers und durch die relativ niedrigen "turn-over-numbers" (<500), d.i. der Quotient aus der Zahl der Mole an gebildetem Produkt pro Mol Katalysator, begrenzt.

Bei W. Reppe, Ann., 601, 81 (1956), sind ferner Zink(II)- und Cadmium (II) verbindungen als Katalysatoren für die Addition von Carbonsäuren und Phenolen an Alkine beschrieben.

Gold (III) verbindungen wie Natriumtetrachloroaurat ($NaAuCl_4$) sind bislang nur an einer Stelle, bei Y. Fukuda, K. Utimoto, J. Org. Chem., 56, 3729 (1991), als Katalysatoren für die Addition von Wasser oder Alkoholen an Alkine beschrieben.

Gold (I) verbindungen wurden offenbar bisher noch nicht als Katalysatoren für die Addition von Nucleophilen an Alkine eingesetzt.

Wie aus der Erläuterung des Standes der Technik ersichtlich ist, existieren bereits zahlreiche Verfahren der Addition von Nucleophilen an Alkine, die jedoch alle ein beschränktes Anwendungsgebiet haben. Die bisher bevorzugten Quecksilberkatalysatoren haben insbesondere den Nachteil der Toxizität und relativ geringer turn-over-numbers, so daß ein hoher Katalysatorverbrauch in Kauf genommen werden mußte bzw. erhebliche Mengen an Nebenprodukten entstanden.

Es bestand daher die Aufgabe, ein möglichst allgemein anwendbares katalytisches Verfahren zur Addition von nucleophilen Agentien an Alkine oder Allene und insbesondere ein solches Verfahren vorzuschlagen, das auch die Addition von schwachen Nucleophilen bzw. die Addition an solche Alkine gestattet, die nicht aktiviert sind und welches die geschilderten Nachteile nicht aufweist.

Diese Aufgabe wurde erfindungsgemäß gelöst durch Bereitstellen eines Verfahrens zur katalytischen Addition von nucleophilen Agentien an Alkine oder Allene unter Bildung von durch das Nucleophil substituierten Alkenen, die gegebenenfalls mit dem Nucleophil weiterreagieren und/oder isomerisieren, das dadurch gekennzeichnet ist, daß man als Katalysator zumindest teilweise in ionisierter Form vorliegende Komplexe des einwertigen Golds verwendet.

Da die Komplexe zumindest teilweise in ionisierter Form vorliegen müssen, wird angenommen, daß die katalytische Wirkung durch ein komplexes Kation der Formel 1

$$L\text{-}Au^{\oplus} \qquad (1)$$

bewirkt wird, worin der Ligand L für die Bausteine

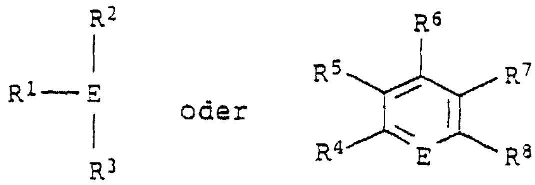

stehen kann und worin

| | |
|---|---|
| $R^1$, $R^2$ und $R^3$ | unabhängig voneinander für gegebenenfalls substituierte aliphatische, cycloaliphatische, aromatische, heteroaromatische oder araliphatische Reste mit 1 bis 30 C-Atomen stehen, die miteinander verbrückt und gegebenenfalls über ein Sauerstoffatom oder ein Stickstoffatom an E gebunden sein können, |
| E | für Phosphor, Arsen oder Antimon steht und |
| $R^4$, $R^5$, $R^6$, $R^7$ und $R^8$ | für Wasserstoff oder einen ggf. substituierten aliphatischen, cycloaliphatischen, aromatischen, heteroaromatischen oder araliphatischen Rest mit 1 bis 30 C-Atomen, eine entsprechende Alkoxygruppe oder Estergruppe oder aber eine Nitrogruppe, Cyanogruppe oder Halogen bedeuten können, wobei |

der Ligand L auch in ein Polymer eingebaut sein kann.

Demgemäß verwendet man als Katalysator vor allem Komplexe der Formel 2

$$L\text{-}Au^{\oplus}\ X^{\ominus} \qquad (2),$$

in der der Ligand L die oben angegebene Bedeutung hat und $X^{\ominus}$ ein Anion, insbesondere ein schwach oder nicht koordinierendes Anion, bedeutet.

Von besonderer Bedeutung ist das erfindungsgemäße Verfahren für Addition von Wasser, Alkoholen mit 1 bis 30 C-Atomen oder Carbonsäuren mit 1 bis 30 C-Atomen an Alkine mit 2 bis 20 C-Atomen oder Allene mit 3 bis 20 C-Atomen in Gegenwart eines Katalysators der Formel 2a

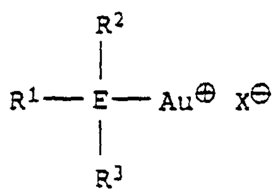

(2a),

in der

| | |
|---|---|
| $R^1$, $R^2$ und $R^3$ | für gegebenenfalls substituierte Alkyl-, Cycloalkyl-, Aryl- oder Aralkylreste mit jeweils 1 bis 30, vorzugsweise 1 bis 10 C-Atomen stehen, die verbrückt und jeweils auch über ein Sauerstoffatom oder ein Stickstoffatom an E gebunden sein können, |
| E | für Arsen, Antimon und vor allem für Phosphor steht und |
| $X^{\ominus}$ | ein Anion, insbesondere ein schwach oder nicht koordinierendes Anion, bedeuten. |

Bevorzugt werden Komplexe der Formel 2a, in der die Reste $R^1$, $R^2$ und $R^3$ für gegebenenfalls substituierte primäre, sekundäre oder tertiäre Alkylreste oder gegebenenfalls substituierte Arylreste wie Pyridyl-, Naphthyl- oder insbesondere Phenylreste oder aber gegebenenfalls substituierte Alkoxy- oder Aryloxyreste stehen.

Als koordinierende Anionen betrachten wir im Rahmen dieser Erfindung z.B. Chlorid, Bromid und Iodid; als schwach koordinierende Anionen z.B. Nitrat, Sulfat, Azid, Cyanat, Sulfonate, wie Tosylat, Methansulfonat, Trifluormethansulfonat, Sulfinate, wie Benzolsulfinat, Alkoholate, wie Methanolat, Ethanolat oder 2,2,2-Trifluorethanolat, Phenolat, Carboxylate, wie Acetat oder Trifluoracetat; und als nicht koordinierende Anionen z.B. Perchlorat, Tetrafluoroborat, Hexafluoroantimonat, Hexafluorophosphat oder Tetraphenylborat.

Mit besonderem Vorteil gelingt das erfindungsgemäße Verfahren, wenn man einen Katalysator der Formel 2a verwendet, in der X ein schwach koordinierendes oder nicht koordinierendes Anion, ausgewählt aus der Gruppe bestehend aus Nitrat, Sulfat, Azid, Sulfonat, Sulfinat, Alkoholat, Phenolat, Carbonxylat, Perchlorat, Terafluoroborat, Hexafluorantimonat, Hexafluorophosphat oder Tetraphenylborat bedeutet.

Die genannten katalytisch wirkenden ionisierten Gold(I)komplexverbindungen sind in der Regel in freier Form nicht beständig und werden deshalb im allgemeinen in situ hergestellt. Dazu bieten sich mehrere an sich bekannte Methoden an (L hat in den folgenden Beispielen immer die oben angegebene Bedeutung):

1. Durch in situ Umsetzung eines stabilen Gold(I)komplexes der Formel 3

$$\text{L-AuX} \qquad (3),$$

in der X einen Rest bedeutet, der zur Bildung eines gut oder schwach koordinierenden Anions befähigt ist, vorzugsweise Nitrat, Sulfat, Sulfonat oder Carboxylat, wobei X kovalent an Au gebunden ist, mit einer Lewissäure, insbesondere Bortrifluorid, vorzugsweise als Etherat oder Methanolat, d.h. vorzugsweise einer Lewissäure, die nur langsam vom Nucleophil angegriffen wird. Dabei wird die Bindung Au-X heterolytisch gespalten.
Gemäß einer Variante ist auch die Herstellung eines $L\text{-}Au^{\oplus}$-Kations im Reaktionsgemisch durch Umsetzung von Oniumsalzen der Formel 5

$$(\text{L-Au})_n Z^{m\oplus}\ {}_m X'^{\ominus} \qquad (5),$$

in der

Z für Sauerstoff oder Schwefel steht, wobei n=3 oder 4 und m=1 bzw. 2 ist, oder für Stickstoff steht, wobei n=4 oder 5 und m=1 bzw. 2 ist, oder für Phosphor steht, wobei n=4, 5 oder 6 und m=1 bzw. 2 bzw. 3 ist, oder für Arsen steht, wobei n=4 und m=1 ist, oder für Kohlenstoff steht, wobei n=4, 5 oder 6 ist und m=0 bzw. 1 bzw. 2 ist, oder für CH steht, wobei n=4 und m=1 ist, oder für RS steht, wobei n=2 und m=1 ist, oder für RN steht, wobei n=2 oder 3 und m=1 bzw. 2 ist, oder aber für RP steht, wobei n=2, 3 oder 4 und m=0 bzw. 1 bzw. 2 ist und wobei R einen niederen aliphatischen oder einen aromatischen Rest bedeutet und

$X'^{\ominus}$ ein schwach oder nicht koordinierendes Anion bedeutet, mit Lewissäuren, insbesondere mit $BF_3$ als Etherat oder Methanolat oder mit Brönsted-Säuren HY, worin Y ein schwach koordinierendes Anion ist.

2. Durch in situ Anionenaustausch zwischen L-Au-Hal (Hal = Cl, Br oder I) und einem Silbersalz, welches ein nicht koordinierendes Anion enthält, und Ausfällung der entstandenen, schwer löslichen Silbersalze AgHal.

3. Durch in situ Umsetzung eines neutralen Gold(I)komplexes der Formel 4

$$\text{L-Au-R'} \qquad (4),$$

in der R' für eine Alkyl-, Cycloalkyl-, Alkenyl-, Alkinyl-, Aralkyl- oder Arylgruppe steht, mit einer Brönsted-Säure HY, wobei Y ein schwach oder nicht koordinierendes Anion bedeutet, oder mit einer Lewis-Säure, insbesondere mit $BF_3$ als Etherat oder Methanolat.

4. Durch Auflösung eines stabilen Gold(I)komplexes der Formel 3

$$\text{L-AuX} \qquad (3),$$

wobei X einen zur Bildung eines Anions befähigten Rest bedeutet, in einem polaren Medium, das die Dissoziation des Komplexes in freie oder solvatisierte Ionen ermöglicht.

Gleichermaßen kommen beliebige weitere Umsetzungen in Betracht, durch die das Kation der Formel 1 in situ generiert werden kann.

Die als Katalysator zu verwendenden ionisierten Gold(I)komplexverbindungen der Formel 2 sowie deren Ausgangsverbindungen der Formeln 3 und 4 sind aus der Literatur z.B. aus D.I. Nichols und A.S. Charleston, J. Chem. Soc. (A) (1969), 2581, US 3,661,959, und Inorganic Synthesis 26, 325 (1989) bekannt, so daß auf diese Stellen verwiesen wird.

Als nucleophile Agentien, oder kurz Nucleophile, kommen für die erfindungsgemäß katalysierte Addition an Alkine oder Allene die an sich bekannten zur Elektronenlieferung befähigten Agentien in Betracht. Insbesondere sind Wasser, Alkohole mit 1 bis 30, vorzugsweise 1 bis 10 C-Atomen, wie Methanol, Ethanol, 2-Propanol oder t-Butanol, Phenole, Carbonsäuren mit 1 bis 20, vorzugsweise 1 bis 10 C-Atomen, wie Ameisensäure, Essigsäure oder Acrylsäure, Thiole, Sulfonsäure, Phosphorsäuren, Halogenwasserstoffe oder auch Verbindungen zu nennen, die eine Kombination der genannten Funktionalitäten aufweisen.

Als Alkine bzw. Allene an das die Nucleophile erfindungsgemäß addiert werden, kommen beliebige Verbindungen, z.B. mit 2 bis 60 bzw. 3 bis 60 C-Atomen in Betracht. Bevorzugt sind jedoch Alkine oder Allene mit 2 bis 8 bzw. 3 bis 8 Kohlenstoffatomen und Alkine mit funktionellen Gruppen. Demgemäß kommen bevorzugt z.B. Alkine (terminale oder interne) von Acetylen bis Octin, Allen, Propargylalkohol, 1-Butin-3-ol oder 2-Butin-1,4-diol als Ausgangsmaterial in Betracht.

Die Addition der nucleophilen Agentien z.B. von Methanol an ein Alkin erfolgt in an sich bekannter Weise nach dem folgenden Schema

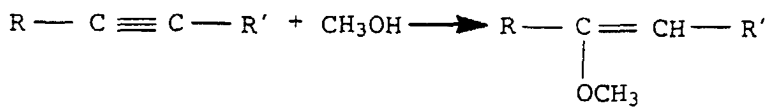

Der entstandene Enoläther lagert in der Regel ein weiteres Molekül Methanol an, so daß die Verbindung

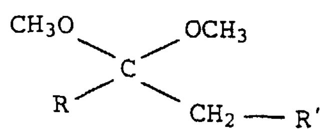

entsteht. Im Falle der Anlagerung von Wasser entsteht aus dem Enol sofort unter Isomerisierung das entsprechende Keton. Gleichermaßen sind Weiterreaktionen durch Ringbildung, wie im folgenden gezeigt, möglich. Diese Reaktionen sind durchweg bekannt und keine Besonderheit des vorliegenden Verfahrens.

In der Folge sind einige erfindungsgemäß katalysierte Additionen als Beispiel der Anwendungsmöglichkeit aufgeführt.

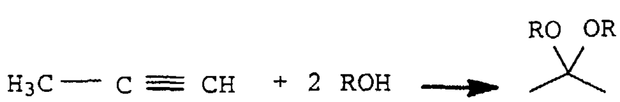

R = Me, Et, iPr

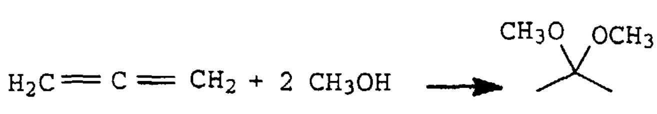

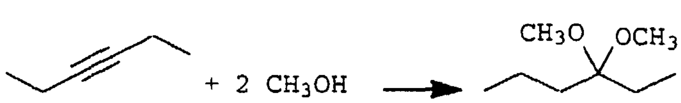

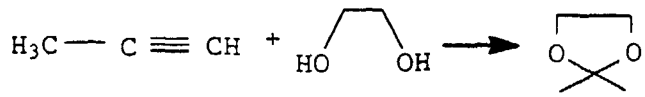

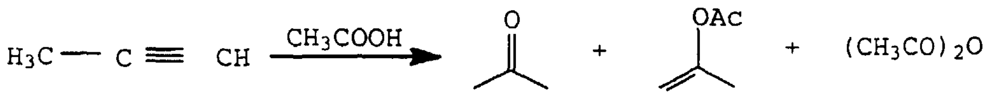


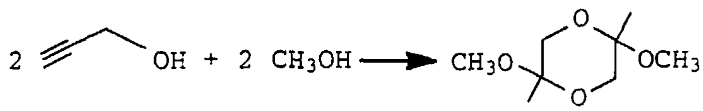

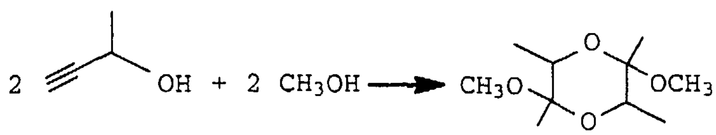

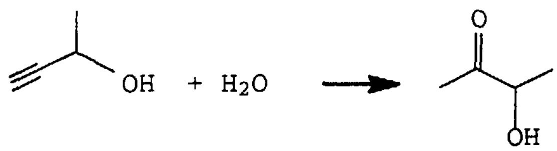

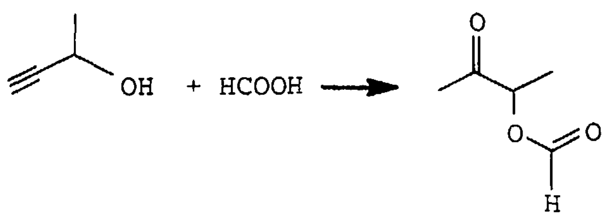

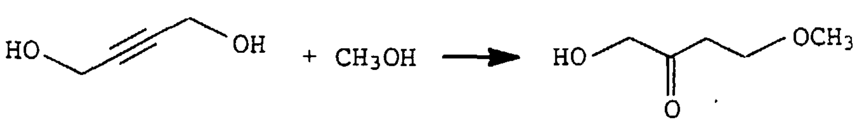

Die erfindungsgemäß katalysierte Addition kann sowohl in Gegenwart wie auch in Abwesenheit eines inerten Lösungsmittels durchgeführt werden. Bevorzugt ist die Durchführung ohne Lösungsmittel, d.h. unter Verwendung des Nucleophils und/oder des Alkins als Reaktionsmedium.

Das molare Verhältnis von Nucleophil zu Alkin bzw. Allen kann zwischen 0,01 und 10000 gewählt werden. Bevorzugt sind Verhältnisse zwischen 0,9 und 100. Besonders bevorzugt sind Verhältnisse zwischen 1 und 5.

Die Reaktionstemperatur beträgt zwischen -30 und +150°C, bevorzugt zwischen 0 und 80°C. Besonders bevorzugt sind Temperaturen zwischen 20 und 60°C.

Die Addition kann bei Normaldruck, vermindertem Druck und erhöhtem Druck durchgeführt werden.

Das molare Verhältnis zwischen Gold(I)komplex, der im Unterschuß eingesetzt wird, und der Summe der Reaktanden kann zwischen 0,1 und $10^{-8}$ gewählt werden. Bevorzugt sind Verhältnisse zwischen 0,01 und $10^{-5}$. Der

Gold(I)komplex wird während der Addition nicht verbraucht und kann aus dem Austrag der Umsetzung nach Abdestillieren des Reaktionsproduktes wiedergewonnen und in die Reaktionsstufe zurückgeführt werden, so daß die Reaktion auch kontinuierlich durchgeführt werden kann. Man erhält ausgezeichnete "turn-over-numbers", die auch bei teilweise -geringem Umsatz im Einfachdurchgang bestehen bleiben, da praktisch keine Nebenprodukte gebildet werden und das Verfahren sich somit durch eine hohe Selektivität auszeichnet. Schließlich ist der Gold(I)komplexkatalysator unter den Reaktionsbedingungen stabil, während z.B. die in der Beschreibungseinleitung genannten Gold(III)komplexe zur Goldabscheidung und damit zur Bildung von einem Goldspiegel an den Reaktoroberflächen neigen.

Beispiele

Die Ausgangsverbindungen für die Katalysatoren wurden jeweils nach literaturbekannten Methoden hergestellt.

Beispiele 1 bis 13

Addition von Methanol an Propin mit L-Au-X + Lewissäure (Methode 1)

In einem Kolben mit Gaseinleitungsrohr, Thermometer und Rückflußkühler wurde Methanol (39,6 g, 1235 mmol) vorgelegt, auf 40°C temperiert und mit Propin vorgesättigt. Der aus Tabelle 1 ersichtliche Gold(I)komplex (0,062 mmol Au) und $BF_3$-Etherat (0,62 mmol) wurden dann rasch nacheinander zugegeben. Während der Reaktionszeit wurde ein schwacher Propinstrom in das Reaktionsgemisch eingeleitet. Die nach 6 Stunden (h) erreichte Konzentration an 2,2-Dimethoxy-propan (DMP) ist in Tabelle 1 angegeben. Außer 2,2-Dimethoxy-propan konnten keine anderen Produkte nachgewiesen werden, d.h. die Reaktion verläuft mit ausgezeichneter Selektivität.

Tabelle 1

| Beispiel | Gold(I)katalysator | DMP-Konzentration [Gew.-%] |
|---|---|---|
| 1 | $Ph_3PAu(NO_3)$ | 42 |
| 2 | $(4\text{-F-Ph})_3PAu(NO_3)$ | 44 |
| 3 | $Ph_2(C_6F_5)PAu(NO_3)$ | 47 |
| 4 | $Et_3PAu(NO_3)$ | 24 |
| 5 | $Ph_3AsAu(NO_3)$ | 32 |
| 6 | $Ph_3PAu(OOCCF_3)$ | 42 |
| 7 | $Ph_3PAu(OOCCF_3)$ *) | 13 |
| 8 | $Ph_3PAuCl$ | 0,7 |
| 9 | $Ph_3PAuI$ | 0,2 |
| 10 | $Ph_3PAuCH_3$ | 40 |
| 11 | $(Ph_3PAu)_3O^+BF_4^-$ | 40 |
| 12 | $(PhO)_3PAu(NO_3)$ | 32 |
| 13 | $(CH_3O)_3PAu(NO_3)$ | 48 |

*) Umsetzung bei 20°C
Ph = Phenyl
Et = Ethyl

Beispiel 14

Addition von Ethanol an Propin mit L-Au-X + Lewissäure (Methode 1)

In einem Kolben mit Gaseinleitungsrohr, Thermometer und Rückflußkühler wurde Ethanol (11,4 g, 245 mmol) vorgelegt, auf 60°C temperiert und mit Propin vorgesättigt. Triphenylphosphangold(I)chlorid (0.062 mmol) und $BF_3$-Etherat (0,62 mmol) wurden dann rasch nacheinander zugegeben. Während der Reaktionszeit wurde ein schwacher Propinstrom in das Reaktionsgemisch eingeleitet. Nach 3 h Reaktionszeit enthielt das Reaktionsgemisch 21,6 Gew.-% 2,2-Diethoxypropan und 0,8 Gew.-% 2-Ethoxypropen. Außer 2,2-Diethoxypropan und 2-Ethoxypropen konnten keine anderen Produkte nachgewiesen werden.

Beispiel 15

Addition von Isopropanol an Propin mit L-Au-R + Brönsted-Säure (Methode 3)

In einem Kolben mit Gaseinleitungsrohr, Thermometer und Rückflußkühler wurde Isopropylalkohol (148,5 g, 2475 mmol) vorgelegt, auf 40°C temperiert und mit Propin vorgesättigt. Triphenylphosphangold(I)methyl (59 mg, 0,124 mmol) und Methansulfonsäure (125 mg, 1,3 mmol) wurden dann rasch nacheinander zugegeben. Während der Reaktionszeit wurde ein schwacher Propinstrom in das Reaktionsgemisch eingeleitet. Nach 6 h enthielt die Reaktionsmischung 2,4 Gew.-% 2-Isopropoxypropen und 1,9 Gew.-% 2,2-Diisopropoxypropan als einzige nachweisbare Produkten.

Beispiel 16

Addition von Allylalkohol an Propin mit L-Au-X + Lewissäure (Methode 1)

In einem Kolben mit Gaseinleitungsrohr, Thermometer und Rückflußkühler wurde Allylalkohol (143,6 g, 2472 mmol) vorgelegt, auf 40°C temperiert und mit Propin vorgesättigt. Triphenylphosphangold(I)nitrat (0,064 g, 0,124 mmol) und $BF_3$-Etherat (1,3 mmol) wurden dann rasch nacheinander zugegeben. Während der Reaktionszeit wurde ein schwacher Propinstrom in das Reaktionsgemisch eingeleitet. Nach 4 h enthielt die Reaktionsmischung 28 % Acetondiallylketal als einziges nachweisbares Produkt.

Beispiel 17

Addition von Essigsäure an Propin mit L-AU-X + Lewissäure (Methode 1)

In einem Kolben mit Gaseinleitungsrohr, Thermometer und Rückflußkühler wurde Essigsäure (9,61 g, 160 mmol) vorgelegt, auf 60°C temperiert und mit Propin vorgesättigt. Triphenylphosphangold(I)-chlorid (0,04 mmol) und $BF_3$-Etherat (0,4 mmol) wurden dann rasch nacheinander zugegeben. Während der Reaktionszeit wurde ein schwacher Propinstrom in das Reaktionsgemisch eingeleitet. Nach 4 h Reaktionszeit enthielt das Reaktionsgemisch 12,9 Gew.-% Aceton. Als Koppelprodukt bildet sich Essigsäureanhydrid.

Beispiel 18

Addition von Essigsäure an Propin mit L-Au-X + Lewissäure (Methode 1)

In einem Kolben mit Gaseinleitungsrohr, Thermometer und Rückflußkühler wurde Essigsäure (74,2 g, 1235 mmol) vorgelegt, auf 40°C temperiert und mit Propin vorgesättigt. Triphenylphosphangold(I)-trifluoracetat (0,062 mmol) und $BF_3$-Etherat (0,62 mmol) wurden dann rasch nacheinander zugegeben. Während der Reaktionszeit wurde ein schwacher Propinstrom in das Reaktionsgemisch eingeleitet. Nach 5 h Reaktionszeit enthielt das Reaktionsgemisch 3,5 Gew.-% Isopropenylacetat und 1,0 Gew.-% Aceton. Als Koppelprodukt konnte Essigsäureanhydrid nachgewiesen werden.

Beispiel 19

Addition von Methanol an Propin mit L-Au-X (Methode 4)

In einem Kolben mit Gaseinleitungsrohr, Thermometer und Rückflußkühler wurde Methanol (7,91 g, 245 mmol) vorgelegt, auf 60°C temperiert und mit Propin vorgesättigt. Triphenylphosphangold(I)-trifluoracetat (0,062 mmol) wurde dann zugegeben. Während der Reaktionszeit wurde ein schwacher Propinstrom in das Reaktionsgemisch eingeleitet. Nach 1 h Reaktionszeit enthielt das Reaktionsgemisch 3,8 Gew.-% 2,2-Dimethoxy-propan. Außer 2,2-Dimethoxy-propan konnten keine anderen Produkte nachgewiesen werden.

Beispiel 20

Addition von Methanol an Propin mit L-Au-X + Ag-Salz (Methode 2)

In einem Kolben mit Gaseinleitungsrohr, Thermometer und Rückflußkühler wurde Methanol (39,55 g, 1235 mmol) vorgelegt, auf 40°C temperiert und mit Propin vorgesättigt. Triphenylphosphangold(I)-chlorid (0,062 mmol) und Silberhexafluoroantimonat wurden dann rasch nacheinander zugegeben. Während der Reaktionszeit wurde ein schwacher Propinstrom in das Reaktionsgemisch eingeleitet. Nach 6 h Reaktionszeit enthielt das Reaktionsgemisch 4,0 Gew.-% 2,2-Dimethoxy-propan. Außer 2,2-Dimethoxy-propan konnten keine anderen Produkte nachgewiesen werden.

Beispiele 21 bis 26

Addition von Methanol an Propin mit L-Au-R' + Brönsted-Säure (Methode 3)

In einem Kolben mit Gaseinleitungsrohr, Thermometer und Rückflußkühler wurde Methanol (79,2 g, 2470 mmol) vorgelegt, auf 40°C temperiert und mit Propin vorgesättigt. Triphenylphosphangold(I)methyl (58,8 mg, 0,124 mmol), die aus Tabelle 2 ersichtliche Menge der aus Tabelle 2 ersichtlichen Säuren wurden dann rasch nacheinander zugegeben. Während der Reaktionszeit wurde ein schwacher Propinstrom in das Reaktionsgemisch eingeleitet. Die nach 6 h Reaktionszeit erreichten Konzentrationen an 2,2-Dimethoxy-propan (2,2-DMP) und 2-Methoxy-propen (2-MP) sind in Tabelle 2 angegeben.

Außer den Wertprodukten 2,2-Dimethoxy-propan und 2-Methoxy-propen konnten nur geringe Mengen an 1,1-Dimethoxy-propan (weniger als 0,3 %) als Nebenprodukt nachgewiesen werden, d.h. die Reaktion verläuft mit ausgezeichneter Selektivität.

Tabelle 2

| Beispiel | Brönsted-Säure | Menge an Säure [Äquiv. bzgl.Au] | 2,2-DMP-Konzentration [Gew.-%] | 2-MP-Konzentration [Gew.-%] |
|---|---|---|---|---|
| 21 | $HBF_4$(54%ig in $Et_2O$) | 10 | 44 | 1 |
| 22 | $CH_3SO_3H$ | 2 | 33 | 0,5 |
| 23 | $CH_3SO_3H$ | 5 | 45 | 2 |
| 24 | $CH_3SO_3H$ | 10 | 52 | 1 |
| 25 | $CH_3SO_3H$ | 25 | 53 | 10 |
| 26 | $CH_3SO_3H$ | 100 | 52 | 21 |

Beispiel 27

Addition von Methanol an 3-Hexin mit L-Au-X + Lewissäure (Methode 1)

In einem Kolben mit Thermometer und Rückflußkühler wurde Methanol (39,6 g, 1235 mmol) und 3-Hexin (25,32 g, 309 mmol) vorgelegt und auf 40°C temperiert. Triphenylphosphangold(I)nitrat (0,062 mmol) und $BF_3$-Etherat (0,62 mmol) wurden dann rasch nacheinander zugegeben. Nach 22 h enthielt das Reaktionsgemisch 57,7 Gew.-% 3,3-Dimethoxy-hexan, 2,2 Gew.-% Hexan-3-on und 5,6 Gew.-% eines Isomerengemisches aus 3-Methoxy-hexen. Außer den genannten Produkten konnten keine anderen nachgewiesen werden.

Beispiel 28

Addition von Methanol an Propargylalkohol mit L-Au-R + Brönsted-Säure (Methode 3)

In einem Dreihalskolben mit Thermometer und Rückflußkühler wurden Methanol (94,5 g, 2,95 mol) und Propargylalkohol (41,35 g, 0,738 mol) vorgelegt und auf 40°C geheizt. Triphenylphosphangold(I)methyl (3,5 mg, 7,35 µmol) und Methansulfonsäure (17,5 mg, 184 µmol) wurden dann rasch hintereinander zugegeben. Nach 23 h bei 40°C waren ca. 98 % des Propargylalkoholes umgesetzt (dies entspricht 98 000 Turn-Overs). Als Hauptprodukt bildete sich ein Gemisch aus beiden Isomeren von 2,5-Dimethoxy-2,5-dimethyl-1,4-dioxan (im Verhältnis 5:1) zusammen mit kleinen Mengen Hydroxyaceton und Hydroxyacetondimethylketal. Nach teilweisem Abzug des Methanols kristallisierte

das Produkt in Form großer farbloser Kristalle (44,5 g, entsprechend 69 % der Theorie isolierte Ausbeute, F = 126°C, F gemäß Literatur = 126-128°C).

Beispiel 29

Addition von Methanol an Propargylalkohol mit L-Au-X + Lewissäure (Methode 1)

In einem Kolben mit Thermometer und Rückflußkühler wurden Methanol (39,6 g, 1235 mmol) und Propargylalkohol (11,54 g, 206 mmol) vorgelegt und auf 40°C temperiert. Triphenylphosphangold(I)nitrat (0,062 mmol) und $BF_3$-Etherat (0,62 mmol) wurden dann rasch nacheinander zugegeben. Nach 4 h wurde das Reaktionsgemisch auf Raumtemperatur abgekühlt, wobei sich 2,5-Dimethyl-2,5-dimethoxy-1,4-dioxan (9,83 g 55 mmol, 54 % Ausbeute) als farbloses Pulver abschied (Smp.: 126 bis 128°C, Lit.: 127°C).

Beispiel 30

Addition von Methanol an 2-Butin-1,4-diol mit L-Au-X + Lewissäure (Methode 1)

In einem Kolben mit Thermometer und Rückflußkühler wurden Methanol (39,6 g, 1235 mmol) und 2-Butin-1,4-diol (17,73 g, 206 mmol) vorgelegt und auf 40°C temperiert. Triphenylphosphangold(I)nitrat (0,062 mmol) und $BF_3$-Etherat (0,62 mmol) wurden dann rasch nacheinander zugegeben. Nach 6 h war das Butindiol vollständig umgesetzt. Als Produkte bildeten sich 4-Methoxy-2-keto-butan-1-ol (90 % Ausbeute), Hydroxymethylvinylketon (5 % Ausbeute) und 2-Keto-butan-1,4-diol (5 % Ausbeute).

Beispiel 31

Addition von Methanol an 2-Butin-1,4-diol mit L-Au-R + Brönsted-Säure (Methode 3)

In einem Dreihalskolben mit Thermometer und Rückflußkühler wurden Methanol (154 g, 4,8 mol) und 2-Butin-1,4-diol (103,2 g, 1,2 mol) vorgelegt und auf 40°C temperiert. Triphenylphosphangold(I)methyl (102,6 mg, 216 µmol) und Methansulfonsäure (0,72 g, 7,2 mmol) wurden dann rasch hintereinander zugegeben. Nach 24 h bei 40°C waren ca. 99 % des 2-Butin-1,4-diols umgesetzt (dies entspricht 5500 Turn-Overs). Die Methansulfonsäure wurde dann durch Zugabe von Natriummethanolat neutralisiert und das Methanol anschließend am Rotationsverdampfer abgezogen. Der Rückstand (gelbliche Flüssigkeit, 127 g, entsprechend ca. 80 % Ausbeute) bestand laut GC zu mehr als 90 % aus 1-Hydroxy-4-methoxy-butan-2-on. Das Produkt kann nur mit großen Verlusten destilliert werden (Kp.: 56-57°C / 4 mbar).

Beispiel 32

Addition von Wasser an 1-Butin-3-ol mit L-Au-X + Lewissäure (Methode 1)

In einem Kolben mit Thermometer und Rückflußkühler wurden 50 g einer 55 %igen wäßrigen Lösung von 1-Butin-3-ol (393 mmol Butinol, 1250 mmol Wasser) vorgelegt und auf 60°C temperiert. Triphenylphosphangold(I)nitrat (0,062 mmol) und $BF_3$-Etherat (0,62 mmol) wurden dann rasch nacheinander zugegeben. Nach 5 h hatte sich 3-Hydroxy-butan-2-on (Acetoin, Ausbeute ca. 6 %) als einziges Produkt gebildet.

Beispiel 33

Addition von Wasser an Propargylalkohol mit L-Au-R + Brönsted-Säure (Methode 3)

In einem Dreihalskolben mit Thermometer und Rückflußkühler wurden Propargylalkohol (41,4 g, 0,74 mol) und Triphenylphosphangold(I)methyl (3,5 mg, 74 µmol) vorgelegt und auf 80°C temperiert. Methansulfonsäure (472 µl, 7,35 mmol) in Wasser (53,2 g, 2,95 mol) gelöst wurde dann zugegeben. Nach 1,5 h bei 80°C enthielt die Lösung 1,5 Gew.-% Hydroxyaceton als einigem Produkt.

Beispiel 34

Addition von Methanol an Butin-3-ol-2 mit $R_3E$-Au-X + Lewissäure (Methode 1)

In einem Kolben mit Thermometer und Rückflußkühler wurden Methanol (39,6 g, 1235 mmol) und wasserfreies 1-Butin-3-ol (23,8 g, 309 mmol) vorgelegt und auf 60°C temperiert. Triphenylphosphangold(I)nitrat (0,062 mmol) und $BF_3$-Etherat (0,62 mmol) wurden dann rasch nacheinander zugegeben. Nach 6 h war das 1-Butin-3-ol zu ca. 50 % umgesetzt. Als Produkte bildeten sich 2,3,4,5-Tetramethyl-2,5-dimethoxy-1,4-dioxan (92 %, als Diastereomerengemisch), 2,3,4,5-Tetramethyl-2-methoxy-1,4-diox-2-en (5 %) und 2,2-Dimethoxy-butan-3-ol (3 %).

Beispiel 35

Addition von Ameisensäure an 1-Butin-3-ol mit L-Au-X + Lewissäure (Methode 1)

In einem Kolben mit Thermometer und Rückflußkühler wurden 1-Butin-3-ol (23,8 g, 309 mmol) und Ameisensäure (28,4 g, 617 mmol) vorgelegt und auf 40°C temperiert. Triphenylphosphangold(I)nitrat (0,031 mmol) und $BF_3$-Etherat (0,62 mmol) wurden dann rasch nacheinander zugegeben. Nach 6 h war das Butinol zu 97 % umgesetzt. Als Produkte bildeten sich 3-Hydroxy-butan-2-on-formiat (89 %) und 3-Hydroxy-butan-2-on (Acetoin, 3 %). Als weiteres Nebenprodukt konnte noch 2,3-Diformyloxy-1-buten (ca. 8 %) nachgewiesen werden.

Beispiel 36

Addition von Ameisensäure an 1-Butin-3-ol mit L-Au-X + Lewissäure (Methode 1)

In einem Kolben mit Tropftrichter, Thermometer und Rückflußkühler wurden Ameisensäure (184 g, 4 mol) und ein Teil des Butinols (3,86 g, 0,05 mol) vorgelegt und auf 50°C temperiert. Triphenylphosphangold(I)nitrat (5,2 mg, 0,01 mmol) und $BF_3$-Etherat (0,1 mmol) wurden dann rasch nacheinander zugegeben. Das restliche Butinol (72,24 g, 0,95 mol) wurde dann innerhalb von 2 h zugetropft. Das Gemisch wurde dann weitere 5 h bei 50°C gerührt. Nach dieser Zeit betrug der Butinolumsatz 82 % (entspricht 82000 Turn-Overs). Die Selektivität zum 2-Hydroxy-butan-3-on-formiat (Acetoinformiat) war 95 %. Das Acetoinformiat konnte durch Destillation des Reaktionsaustrags rein gewonnen werden (Kp.: 53°C / 15 mbar).

Beispiel 37

Addition von Methanol an Acetylen mit L-Au-R + Brönsted-Säure (Methode 3)

In einem Kolben mit Gaseinleitungsrohr, Thermometer und Rückflußkühler wurde Methanol (79,2 g, 2470 mmol) vorgelegt, auf 55°C temperiert und mit Acetylen vorgesättigt. Triphenylphosphangold(I)methyl (58,8 mg, 0,124 mmol) und Methansulfonsäure (12,4 mmol) wurden dann rasch nacheinander zugegeben. Während der Reaktionszeit wurde ein schwacher Acetylenstrom in das Reaktionsgemisch eingeleitet. Nach 4 h Reaktionszeit enthielt die Reaktionslösung 1,5 Gew.-% 1,1-Dimethoxy-ethan als einzigem Produkt.

Beispiel 38

Addition von Methanol an Phenylacetylen mit L-Au-R + Brönsted-Säure (Methode 3)

In einem Kolben mit Thermometer und Rückflußkühler wurden Methanol (8,01 g, 250 mmol) und Phenylacetylen (6,38 g, 62,5 mmol) vorgelegt und auf 40°C temperiert. Triphenylphosphangold(I)methyl (14,8 mg, 31,2 µmol) und Methansulfonsäure (40,6 µl, 625 µmol) wurden dann rasch nacheinander zugegeben. Nach 8 h Reaktionszeit war das Phenylacetylen zu 99 % umgesetzt. Als Hauptprodukte bildeten sich α-Methoxy-styrol (Selektivität (S) = 61 %) und Acetophenondimethylketal (S = 20 %). Durch nachfolgende säurekatalysierte Umsetzung des α-Methoxy-styrols bildeten sich als Nebenprodukte 1-Methoxy-1,3-diphenyl-butadien (als 1:1 cis/trans-Gemisch, S = 13 %), Acetophenon (S = 4 %) und 1,3,5-Triphenyl-benzol (S = 2 %).

Beispiel 39

Addition von Methanol an Diphenylacetylen (Tolan) mit L-Au-R + Brönsted-Säure (Methode 3)

In einem Kolben mit Thermometer und Rückflußkühler wurden Methanol (108,1 g, 3,37 mol) und Tolan (15,0 g, 84,3 mmol) vorgelegt und auf 40°C temperiert. Triphenylphosphangold(I)methyl (20,0 mg, 42 µmol) und Methansulfonsäure (54,8 µl, 840 µmol) wurden dann rasch nacheinander zugegeben. Nach 20 h Reaktionszeit war das Tolan zu 35 % umgesetzt. Als einziges Produkt bildete sich 1-Methoxy-1,2-diphenyl-ethen (als E/Z-Gemisch, ca. 1:2).

## Patentansprüche

1. Verfahren zur katalytischen Addition von nucleophilen Agentien an Alkine oder Allene unter Bildung von durch das Nucleophil substituierten Alkenen, die gegebenenfalls mit dem Nucleophil weiterreagieren und/oder isomerisieren, dadurch gekennzeichnet, daß man als Katalysator zumindest teilweise in ionisierter Form vorliegende Komplexe des einwertigen Golds verwendet.

2. Verfahren gemäß Anspruch 1, dadurch gekennzeichnet, daß man einen Katalysator verwendet, der ein komplexes Kation der Formel 1

$$L\text{-}Au^{\oplus} \qquad (1)$$

aufweist, worin der Ligand L für die Bausteine

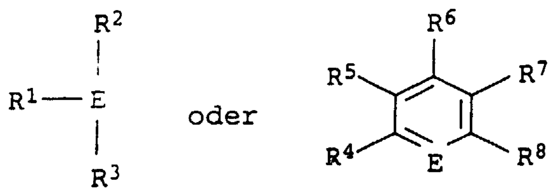
oder

stehen kann und worin

| | |
|---|---|
| $R^1$, $R^2$ und $R^3$ | unabhängig voneinander für gegebenenfalls substituierte aliphatische, cycloaliphatische, aromatische, heteroaromatische oder araliphatische Reste mit 1 bis 30 C-Atomen stehen, die miteinander verbrückt und gegebenenfalls über ein Sauerstoffatom oder ein Stickstoffatom an E gebunden sein können, |
| E | für Phosphor, Arsen oder Antimon steht und |
| $R^4$, $R^5$, $R^6$, $R^7$ und $R^8$ | für Wasserstoff oder einen ggf. substituierten aliphatischen, cycloaliphatischen, aromatischen, heteroaromatischen oder araliphatischen Rest mit 1 bis 30 C-Atomen, eine entsprechende Alkoxygruppe oder Estergruppe oder aber eine Nitrogruppe, Cyanogruppe oder Halogen bedeuten können, wobei |

der Ligand L auch in ein Polymer eingebaut sein kann.

3. Verfahren gemäß Anspruch 1, dadurch gekennzeichnet, daß man Nucleophile an Alkine mit 2 bis 60 C-Atomen oder an Allene mit 3 bis 60 C-Atomen in Gegenwart eines Katalysators der Formel 2

$$L\text{-}Au^{\oplus}\, X^{\ominus} \qquad (2)$$

addiert, in der L die in Anspruch 2 angegebenen Bedeutungen hat und $X^{\ominus}$ ein Anion bedeutet.

**4.** Verfahren gemäß Anspruch 1, dadurch gekennzeichnet, daß man Wasser, Alkohole mit 1 bis 30 C-Atomen oder Carbonsäuren mit 1 bis 30 C-Atomen an Alkine mit 2 bis 20 C-Atomen oder Allene mit 3 bis 20 C-Atomen in Gegenwart eines Katalysators der Formel 2a addiert

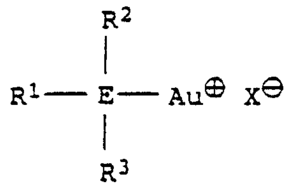 (2a),

in der

$R^1$, $R^2$ und $R^3$ für Alkyl- oder Arylreste mit 1 bis 10 C-Atomen stehen, die gegebenenfalls substituiert und/oder über ein Sauerstoffatom an E gebunden sein können,

E Phosphor bedeutet und

$X^{\ominus}$ ein schwach oder nicht koordinierendes Anion bedeutet.

**5.** Verfahren gemäß Anspruch 4, dadurch gekennzeichnet, daß man einen Katalysator der Formel 2a verwendet, in der X ein schwach koordinierendes oder nicht koordinierendes Anion, ausgewählt aus der Gruppe bestehend aus Nitrat, Sulfat, Azid, Sulfonat, Sulfinat, Alkoholat, Phenolat, Carboxylat, Perchlorat, Tetrafluoroborat, Hexafluoroantimonat, Hexafluorophosphat oder Tetraphenylborat bedeutet.

**6.** Verfahren gemäß Anspruch 1, dadurch gekennzeichnet, daß man den Katalysator in situ im Reaktionsgemisch erzeugt.

**7.** Verfahren gemäß Anspruch 1, dadurch gekennzeichnet, daß man den Katalysator durch Umsetzung von neutralen Gold(I)komplexen der Formel 3

$$L\text{-}AuX \qquad (3),$$

in der der Ligand L die in Anspruch 2 angegebene Bedeutung hat und X einen zur Bildung eines Anions befähigten Rest bedeutet, mit einer Lewis-Säure in situ erzeugt.

**8.** Verfahren gemäß Anspruch 1, dadurch gekennzeichnet, daß man den Katalysator durch Umsetzung von neutralen Gold(I)komplexen der Formel 3

$$L\text{-}AuX \qquad (3),$$

in der der Ligand L die in Anspruch 2 angegebene Bedeutung hat und X Cl, Br, oder J bedeutet, mit einem Silbersalz, welches ein nicht koordinierendes Anion enthält, in situ erzeugt.

**9.** Verfahren gemäß Anspruch 1, dadurch gekennzeichnet, daß man den Katalysator durch Umsetzung eines neutralen Gold(I)komplexes der Formel 4

$$L\text{-}Au\text{-}R' \qquad (4),$$

in der der Ligand L die in Anspruch 2 angegebene Bedeutung hat und R' eine Alkyl-, Cycloalkyl-, Alkenyl-, Alkinyl-, Aralkyl- oder Arylgruppe bedeutet, mit einer Brönsted-Säure HY, wobei Y für ein schwach oder nicht koordinierendes Anion steht oder mit einer Lewis-Säure, in situ erzeugt.

**10.** Verfahren gemäß Anspruch 1, dadurch gekennzeichnet, daß man den Katalysator durch Dissoziation eines stabilen Gold(I)komplexes der Formel 3

$$\text{L-AuX} \qquad (3),$$

in der der Ligand L die in Anspruch 2 angegebene Bedeutung hat und X einen zur Bildung eines Anions befähigten Rest darstellt, durch Einwirkung eines polaren Mediums erzeugt.

## Claims

**1.** A process for the catalytic addition of nucleophilic agents to alkynes or allenes to form alkenes substituted by the nucleophile which may further react with the nucleophile and/or isomerize, which comprises using a catalyst comprising a wholly or partly ionized complex of univalent gold.

**2.** A process as claimed in claim 1, wherein the catalyst used comprises a complex cation of the formula 1

$$\text{L-Au}^{\oplus} \qquad (1)$$

where the ligand L can represent the building blocks

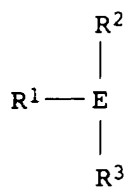

or

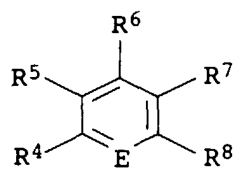

and where

| | |
|---|---|
| $R^1$, $R^2$ and $R^3$ | independently of the others represent substituted or unsubstituted aliphatic, cycloaliphatic, aromatic, heteroaromatic or araliphatic radicals having from 1 to 30 carbon atoms which may be bridged and may optionally be attached to E via an oxygen atom or via a nitrogen atom, |
| E | is phosphorus, arsenic or antimony, and |
| $R^4$, $R^5$, $R^6$, $R^7$ and $R^8$ | are each hydrogen or a substituted or unsubstituted aliphatic, cycloaliphatic, aromatic, heteroaromatic or araliphatic radical having from 1 to 30 carbon atoms, a corresponding alkoxy group or ester group or alternatively a nitro group, cyano group or halogen, and |

the ligand L can also have been incorporated into a polymer.

**3.** A process as claimed in claim 1, wherein nucleophiles are added to alkynes having from 2 to 60 carbon atoms or to allenes having from 3 to 60 carbon atoms in the presence of a catalyst of the formula 2

$$\text{L-Au}^{\oplus}\,\text{X}^{\ominus} \qquad (2)$$

where L is as defined in claim 2 and $X^{\ominus}$ is an anion.

**4.** A process as claimed in claim 1, wherein water, alcohols having from 1 to 30 carbon atoms or carboxylic acids having from 1 to 30 carbon atoms are added to alkynes having from 2 to 20 carbon atoms or allenes having from 3 to 20 carbon atoms in the presence of a catalyst of the formula 2a

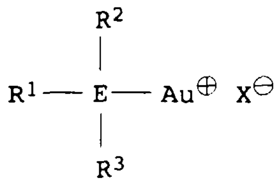 (2a),

where

R¹, R² and R³ are alkyl or aryl radicals having from 1 to 10 carbon atoms, which may be substituted or unsubstituted and may each be attached to E via an oxygen atom,

E is phosphorus, and

$X^{\ominus}$ is a weakly coordinating or noncoordinating anion.

**5.** A process as claimed in claim 4, wherein the catalyst used has the formula 2a where X is a weakly coordinating or noncoordinating anion selected from the group consisting of nitrate, sulfate, azide, sulfonate, sulfinate, alcoholate, phenolate, carboxylate, perchlorate, tetrafluoroborate, hexafluoroantimonate, hexafluorophosphate and tetraphenylborate.

**6.** A process as claimed in claim 1, wherein the catalyst is formed in situ in the reaction mixture.

**7.** A process as claimed in claim 1, wherein the catalyst is formed in situ by reacting a neutral gold(I) complex of the formula 3

$$L\text{-}AuX \qquad (3),$$

where the ligand L is as defined in claim 2 and X is a radical capable of forming an anion, with a Lewis acid.

**8.** A process as claimed in claim 1, wherein the catalyst is formed in situ by reacting a neutral gold(I) complex of the formula 3

$$L\text{-}AuX \qquad (3),$$

where the ligand L is as defined in claim 2 and X is a Cl, Br or I, with a silver salt containing a noncoordinating anion.

**9.** A process as claimed in claim 1, wherein the catalyst is formed in situ by reacting a neutral gold(I) complex of the formula 4

$$L\text{-}Au\text{-}R' \qquad (4),$$

where the ligand L is as defined in claim 2 and R' is alkyl, cycloalkyl, alkenyl, alkynyl, aralkyl or aryl, with a Brönsted acid HY, where Y is a weakly coordinating or noncoordinating anion, or with a Lewis acid.

**10.** A process as claimed in claim 1, wherein the catalyst is formed by dissociation of a stable gold(I) complex of the formula 3

$$L\text{-}AuX \qquad (3),$$

where the ligand L is as defined in claim 2 and X is a radical capable of forming an anion, by the action of a polar medium.

## Revendications

**1.** Procédé pour fixer par addition catalytique des agents nucléophiles sur des alcynes ou des allènes avec formation d'alcènes substitués par le nucléophile qui, le cas échéant, continuent de réagir avec le nucléophile et/ou s'isomérisent, caractérisé par le fait que l'on utilise en tant que catalyseurs des complexes de l'or monovalent en partie au moins à l'état ionisé.

**2.** Procédé selon la revendication 1, caractérisé par le fait que l'on utilise un catalyseur à cation complexe de formule 1

$$L\text{-}Au^{\oplus} \qquad (1)$$

dans laquelle le ligand L peut consister en l'un des motifs de structure

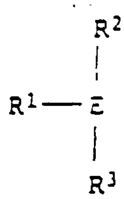

ou

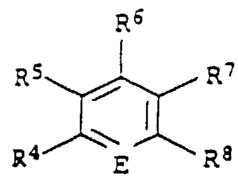

dans lesquels

$R^1$, $R^2$ et $R^3$ représentent chacun, indépendamment les uns des autres, des radicaux aliphatiques, cycloaliphatiques, aromatiques, hétéroaromatiques ou araliphatiques en C1-C30 éventuellement substitués qui peuvent être reliés entre eux par des ponts et le cas échéant reliés à E par l'intermédiaire d'un atome d'oxygène ou d'un atome d'azote,

E représente le phosphore, l'arsenic ou l'antimoine et

$R^4$, $R^5$, $R^6$, $R^7$ et $R^8$ représentent chacun l'hydrogène ou un radical aliphatique, cycloaliphatique, aromatique, hétéroaromatique ou araliphatique en C1-C30, éventuellement substitué, un groupe alcoxy correspondant ou un groupe ester ou encore un groupe nitro, un groupe cyano ou un halogène,

le ligand L pouvant également être inclus dans un polymère.

**3.** Procédé selon la revendication 1, caractérisé par le fait que l'on fixe des nucléophiles par addition sur des alcynes en C2-C60 ou sur des allènes en C3-C60 en présence d'un catalyseur de formule 2

$$L\text{-}Au^{\oplus}\ X^{\ominus} \qquad (2)$$

dans laquelle L a les significations indiquées dans la revendication 2 et $X^{\ominus}$ représente un anion.

**4.** Procédé selon la revendication 1, caractérisé par le fait que l'on fixe par addition de l'eau, des alcools en C1-C30 ou des acides carboxyliques en C1-C30 sur des alcynes en C2-C20 ou des allènes en C3-C20 en présence d'un catalyseur de formule 2a

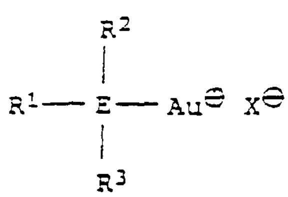

(2a) ,

dans laquelle

$R^1$, $R^2$ et $R^3$ représentent des groupes alkyle ou aryle en C1-C10 éventuellement substitués et/ou qui peuvent être reliés à E par l'intermédiaire d'un atome d'oxygène,

E représente le phosphore et

$X^{\ominus}$ représente un anion faiblement coordinant ou non coordinant.

**5.** Procédé selon la revendication 4, caractérisé par le fait que l'on utilise un catalyseur de formule 2a dans laquelle X représente un anion faiblement coordinant ou non coordinant choisi parmi les anions nitrate, sulfate, azothydrate, sulfonate, sulfinate, alcoolate, phénolate, carboxylate, perchlorate, tétrafluoroborate, hexafluoroantimoniate, hexafluorophosphate ou tétraphénylborate.

**6.** Procédé selon la revendication 1, caractérisé par le fait que l'on forme le catalyseur in situ dans le mélange de réaction.

**7.** Procédé selon la revendication 1, caractérisé par le fait que l'on forme le catalyseur in situ par réaction de complexes neutres de l'or-I répondant à la formule 3

$$L\text{-}AuX \qquad (3),$$

dans laquelle L représente un ligand tel que défini dans la revendication 2 et X est un radical capable de former un anion, avec un acide de Lewis.

**8.** Procédé selon la revendication 1, caractérisé par le fait que l'on forme le catalyseur in situ par réaction de complexes neutres d'or-I répondant à la formule 3

$$L\text{-}AuX \qquad (3),$$

dans laquelle L représente un ligand tel que défini dans la revendication 2 et X représente Cl, Br ou I, avec un sel d'argent contenant un anion non coordinant.

**9.** Procédé selon la revendication 1, caractérisé par le fait que l'on forme le catalyseur in situ par réaction d'un complexe neutre de l'or-I répondant à la formule 4

$$L\text{-}Au\text{-}R' \qquad (4),$$

dans laquelle L représente un ligand tel que défini dans la revendication 2 et R' représente un groupe alkyle, cycloalkyle, alcényle, alcynyle, aralkyle ou aryle, avec un acide de Brönsted HY, Y représentant un anion faiblement coordinant ou non coordinant, ou avec un acide de Lewis.

**10.** Procédé selon la revendication 1, caractérisé par le fait que l'on forme le catalyseur par dissociation d'un complexe stable de l'or-I répondant à la formule 3

$$L\text{-}AuX \qquad (3),$$

dans laquelle L représente un ligand tel que défini dans la revendication 2 et X représente un groupe capable de former un anion, sous l'action d'un milieu polaire.